# EUROPEAN PATENT SPECIFICATION

(11) **EP 1 509 128 B1**
(45) Date of publication and mention of the grant of the patent: **20.08.2008**
(21) Application number: 03749968.8
(22) Date of filing: 05.05.2003
(51) Int. Cl.: A61B 5/024, D04B 1/24

(54) **TEXTILE ARTICLE HAVING ELECTRICALLY CONDUCTIVE PORTIONS AND METHOD FOR PRODUCING THE SAME**
TEXTILER ARTIKEL MIT ELEKTRISCH LEITENDEN ABSCHNITTEN UND VERFAHREN ZU SEINER HERSTELLUNG
ARTICLE TEXTILE COMPORTANT DES PARTIES ELECTRO-CONDUCTRICES ET SON PROCEDE DE PRODUCTION

(30) Priority: 14.05.2002 GB 0210888
(43) Date of publication of application: 02.03.2005
(73) Proprietor: Koninklijke Philips Electronics N.V., 5621 BA Eindhoven (NL)
(72) Inventor: VALENTINE, Warren B. Philips Intel. Prop.& Stand., Redhill, Surrey RH1 5HA (GB); TILBURY, Nancy, A. Philips Intel. Prop. & Stand., Redhill, Surrey RH1 5HA (GB); WAGNER, Philippa, C. Philips Intel. Prop. & Stand., Redhill, Surrey RH1 5HA (GB); ARCHER, Isabel Philips Intel. Prop. & Standards, Redhill, Surrey RH1 5HA (GB); MAMA, Kyriakos Philips Intel. Property & Standards, Redhill, Surrey RH1 5HA (GB)
(74) Representative: Damen, Daniel Martijn
(86) International application number: PCT/IB2003/001780
(87) International publication number: WO 2003/094717

(56) References cited:
- EP-A- 1 176 242
- EP-A- 1 442 168
- WO-A-01/02052
- WO-A-02/071935
- DE-U- 29 623 022

## Description

The present invention relates to textile articles, in particular to textile articles having electrically conductive portions, and methods for providing such textile articles.

The integration into garments or the like of electrical or electronic components is of interest to the so called "wearable electronics" community and for health monitoring and treatment applications. Providing wiring in a garment is not necessarily the most elegant solution since the provision of such wiring can detract from the look or hang of a garment. Furthermore, providing wiring and associated connectors in garments or the like usually involves techniques which are not common place in the garment instruction industry.

One approach to address some of the above concerns is to include conductive yarns in the textiles of a garment itself to provide conductive tracking and the like. Such tracking usually needs to be insulated or hidden in particular regions and this may be done by providing further layers of textile in a traditional "cut and sew" approach. Such an approach can modify the look or hang of the garment in an undesirable way and leads to further labour and cost during production.

An example of such an approach is disclosed in International Patent Application publication WO/0102052, which relates to a garment, adapted to be used as a medical electrode. The garment comprises a tubular body, which comprises at least two different zones. One of these zones is an electrically conductive zone, to be used as the electrode surface of the medical electrode. Another zone is an elastic zone, which comprises electrically non-conductive yarns. This elastic zone assures the position of the electrically conductive zone on the corpus on which the medical electrode is to be used.

It is an object of the present invention to at least partially address the above mentioned problems.

In accordance with the first aspect of the present invention there is provided a textile article comprising two or more at least partially overlapping layers, a first one of the layers having a portion which includes electrically

It is an object of the present invention to at least partially address the above mentioned problems.

In accordance with the first aspect of the present invention there is provided a textile article comprising two or more at least partially overlapping layers, a first one of the layers having a portion which includes electrically conductive material and a second one of the layers having a portion which includes electrically insulative material, wherein the portion including electrically insulative material is disposed adjacent the portion including the electrically conductive material to at least partially cover and thus serve to electrically insulate the portion including electrically conductive material characterised in that the first one and second one of the layers are formed of a common integral component folded to provide an overlapping region and that the portion which includes electrically conductive material forms an electrode for detecting electrical signals of a wearer.

The first and second one of the layers may be of knitted construction. In this case, the layers may be the product of a circular knitting technique. Where a circular knitting technique is employed, the first one and the second one of the layers may be formed from a component which is of generally tubular shape, the component being subsequently folded to tuck one portion of the component within another portion of the component to provide, respectively, an inner tubular section forming the first one layer and an outer tubular section forming the second one layer.

Thus it is possible to provide an article having conductive tracking or electrodes included in a one knitted layer to be insulated by another adjacent layer merely by knitting a component to the required pattern and performing minimal post knitting work. Such textile articles can potentially be produced with a lower manufacturing cost than would be the case for articles produced without the benefit of the present invention. Such textile articles can also benefit in terms of look and hang because there is a reduced need to sew together multiple components and therefore allows a simple, comfortable garment with no or few seams to be produced.

These and other aspects of the present invention appear in the appended claims which are incorporated herein by reference and to which the reader is now referred.

The present invention will now be described with reference to the figures of the accompanying drawings in which:
Figure 1 is a perspective view of a textile construction for forming a first embodiment of a textile article of the present invention;
Figure 2 is a perspective view of a first embodiment of a textile article of the present invention;
Figure 3 is a cross sectional view of a portion of textile material;
Figure 4 is the material of Figure 3 folded once;
Figure 5 is the material of Figure 3 folded twice.

It should be noted that the drawings are diagrammatic and not drawn to scale. Relative dimensions and proportions of parts of the figures have been shown exaggerated or reduced in size for the sake of clarity and convenience in the drawings. The same reference signs are generally used to refer to corresponding or similar features in a different embodiment.

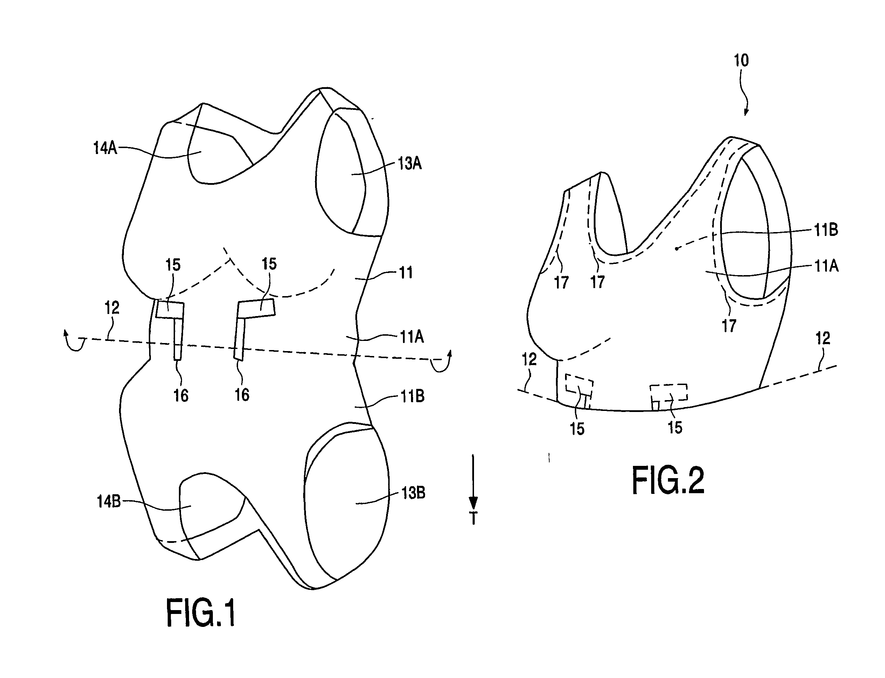

Referring to Figure 1, a textile article 10 is provided by using a circular knitting process (often referred to as a seamless knit) to form a construction 11 that is of generally tubular or cylindrical shape in the direction denoted T. The construction 11 is formed as one piece and comprises a first part 11 a and a second part 11 b, the shape of the second part 11 b being generally a mirror image of the shape of the first part 11 a when viewed with respect to a dividing line 12 disposed generally half way along the tubular construction. The textile article 10 of this example is a bra or ladies top, and although the construction is described as being generally tubular, the diameter of the construction can be varied as required at different areas by selectively using different stitch construction and / or by introducing resiliently extendible yarns (such as elastaine). The knit pattern may be controlled to produce openings such as 13a, 13b, 14a, 14b as will be appreciated by the person skilled in the art. The knitted construction 11 is produced from one or more yarns which are generally electrically insulative. However, two regions 15 of the knitted construction 11 include electrically conductive yam to render these regions electrically conductive. These regions serve as electrodes for detecting electrical signals of a wearer associated with their pulse. The regions 15 are provided in a part of the knitted construction such that when the garment 10 is worn the electrically conductive regions each contact the wearers skin at a location below each bust towards the lower part of the rib cage. In practice, the electrodes may contact the wearer's skin at a other locations, providing appropriate electrical signals may still be detected, as will be known to the person skilled in the art.

The electrically conductive regions are an integral part of the knitted construction 11 and are defined by splicing in conductive yarn as required. Splicing refers to the selective introduction of yarns in a region of the knit. Each conductive electrode forming region 15 is provided with electrically connected knitted conductive tracking 16 leading therefrom allowing signals detected by electrode forming regions 15 to be communicated to means for further processing of the signal, such as amplification and transmission, as the case may be. Conductive tracking 16 may be provided in the same or similar manner as that employed for producing the conductive electrode forming regions 15.

Once the knitted construction stage 11 has been completed, the second part 11 b is lifted up to fold the knitted construction at dividing line 12, such that the second part 11b forms an outer layer of article 10 while the first part 11 a forms an inner layer of article 10. Opening 13a is aligned with opening 13b to form a first armhole and opening 14a is aligned with opening 14b to form a second armhole. The inner and outer layer formed by first part 11 a and second part 11 b may be held in position with respect to each other by stitching, shown in Figure 2 as broken lines denoted 17. However, this adjoining is not mandatory and other techniques for holding the inner and outer layers in the required relationship with respect to each other are possible.

Silicon may be arranged around the periphery of the regions 15 of electrically conductive yarn to provide additional grip between the garment in this region and a wearer's skin. Such additional grip reduces movement of the sensing electrodes in the electrode forming regions 15 and the skin, with a reduction in movement resulting in a reduction of movement induced electrical noise appearing in signals provided by electrodes. It has been observed that an amount of electrically conductive moisture present in the vicinity of the centre of electrodes, in this case in the form of the knitted regions 15, improves the sensing of electrical signals associated with user pulse. Therefore silicon may be provided so as to encourage perspiration in the vicinity of the sensing electrodes, or retain moisture in those electrode regions. In one arrangement, silicon is deposited on the inner layer of the bra to form channels, such that during use perspiration of a user is guided by the channels into the vicinity of the electrodes. Silicon may be deposited on the inner layer of the bra to form channels, such that during use perspiration of a user is guided by the channels away from the vicinity of the tracking. In one specific arrangement, during use, perspiration produced in the area under the bust is guided by plurality of channels under the influence of gravity towards the electrodes. The channels are formed by a plurality of silicon ribs positioned on the inner surface of inner layer 11a, pressing against a user's skin.

The garment may be given further shape inducing treatment through application of so called "boarding" or "shaping" where the garment is held in the shape required while heat is applied; the heat causes the garment to retain the shape. Other garment construction techniques may also be applied as will be apparent to the persons skilled in the art.

The formation of inner and outer layers by first part 11a and second part 11 b, respectively results in the conductive electrode forming regions 15 becoming part of the inner layer, but shielded from external view and contact by the outer layer. This is indicated in Figure 2 by showing the conductive electrode forming regions 15 with broken lines.

It will be apparent to the person skilled in the art the practice of producing an item by forming a textile construction with selective conductive and insulative regions and then folding that construction accordingly to allow the conductive regions to be selectively exposed or shielded allows a large number of arrangements to be produced. For example, a layer containing electrically conductive tracking can be insulated from a person's skin by providing an insulating layer disposed to hold a conductive tracking away from the skin. This insulating layer may be provided in a printing stage which deposits electrically insulating material. Furthermore, electrically conductive regions may be provided in a printing stage, which deposits electrically conductive material.

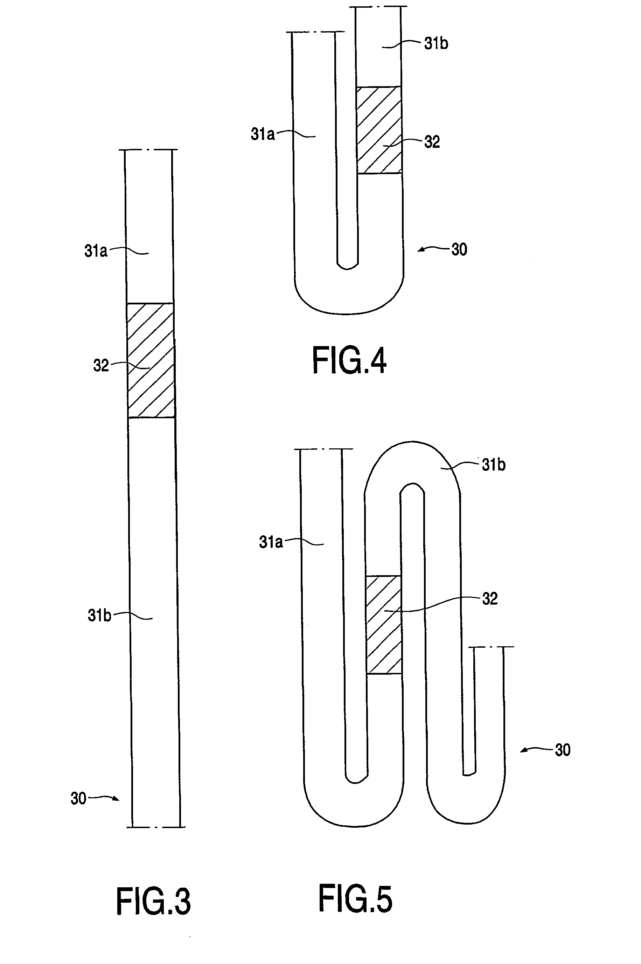

The above example describes a textile item produced by folding a construction once to produce inner and outer layers. This is illustrated schematically with reference to Figures 3 and 4. A portion of textile material 30 is produced with an electrically conductive region 32 surrounded by electrically insulating regions 31a, 31b. This may be folded to a folded state as shown in Figure 4 such that portion 31a shields electrically conducted portion 32 on one side. However, the present invention is not limited to folding the textile construction only once; one or more folds may be formed. This is illustrated with reference to Figure 5 where the portion of textile material 30 is folded twice in serpentine fashion such that first insulating portion 31 a shields a one side of electrically conducted portion 32 and second insulating portion 31b shields the other side of electrically conductive portion 32. Hence electrically conductive portion 32 is completely shielded by electrically insulative portions 31.

Yarns may be monofiliment of multifilament. Insulating regions may comprise a one or more type of yarn. Conductive regions may comprise of one or more type of yarn. Conductive regions may include electrically conductive and electrically insulative yarns. Suitable conductive yam materials include carbon fibre conductive polymers, or polymers otherwise having a conductive property due to their composition. Other suitable conducting materials include insulators such as polyamide coated with conductors, for example coatings of copper, aluminium, gold and silver. Plating techniques include electroless plating. Yarns may be of metal. Suitable insulating materials include nylon, polyamide, acetate, cotton and wool. In fact any suitable textile materials may be used irrespective of whether they are natural or man made providing they can be processed to form an article of the present invention.

The present invention has been described with specific reference to an embodiment in the form of a bra or ladies top. However, this is by example and in fact the textile article may adopt other forms, including a gents top; some other garment, clothing accessory, health monitoring device, health care treatment device, sports training product, wearable computing arrangement or component for use in one or more of those products without departing from the scope of the present invention.

From reading the present disclosure, other modifications will be apparent to persons skilled in the art. Such modifications may involve other features which are already known in the design manufacture and use of textile articles such as garments, clothing accessories, health monitoring or treatment devices, sports training products, wearable computer arrangements and applications thereof, and which may be used instead of or in addition to features already described herein.

## Claims

1. A textile article (10) comprising two or more at least partially overlapping layers, a first one of the layers (11a) having a portion (15, 32) which includes electrically conductive material and a second one of the layers (11b) having a portion (11b, 31) which includes electrically insulative material, wherein the portion (31) including electrically insulative material is disposed adjacent the portion (32) including the electrically conductive material to at least partially cover and thus serve to electrically insulate the portion including electrically conductive material, **characterised in that** the first one (11a) and the second one (11b) of the layers are formed of a common integral component (11) folded to provide an overlapping region and that the portion (32) which includes electrically conductive material forms an electrode (15) for detecting electrical signals of a wearer.

2. A textile article (10) in accordance with claim 1 wherein the first one and the second one of the layers are of knitted construction.

3. A textile article (10) in accordance with claim 2 wherein the layers are the product of a circular knitting technique.

4. A textile article in accordance with claim 3 wherein the first one and the second one of the layers are formed from a component (11) which is of generally tubular shape, the component being subsequently folded to tuck one portion (11a) of the component within another portion (11b) of the component to provide, respectively, an inner tubular section forming the first one layer and an outer tubular section forming the second one layer.

5. A textile article in accordance with any one or more of claims 1 to 4 wherein the portion (32) which includes electrically conductive material forms conductive tracking (16).

6. A textile article in accordance with any one or more of claims 1 to 5 wherein a textile article (10) is one of a garment, clothing accessory, health monitoring device, health care treatment device, sports training product, wearable computing arrangement, or a component for use in one or more of those products.

7. A method of providing a textile article (10) comprising two or more at least partially overlapping layers, said method comprising the steps of:
providing a first layer (11a) having a portion (15, 32) which includes electrically conductive material;
providing a second layer (11b) having a portion which includes electrically insulative material;
arranging said layers such that the portion including the electrically insulative material is disposed adjacent to the portion (32) including the electrically conductive material to at least partially cover and thus serve to electrically insulate the portion including electrically conductive material, **characterised in that** the first one (11a) and the second one (11b) of the layers are formed by folding a common integral component (11) to provide an overlapping region and that the portion (32) which includes electrically conductive material forms an electrode (15) for detecting electrical signals of a wearer.

8. A method according to claim 7 comprising the step of knitting the first one and the second one of the layers thereby providing said layers.

9. A method according to claim 8 wherein knitting is circular knitting.

10. A method according to claim 8 or 9 and further comprising the steps of forming the first one and the second one of the layers from a component (11) which is of generally tubular shape; and folding the components to tuck one portion (11a) of the component within another portion (11b) of the component to provide, respectively, an inner tubular section forming the first one layer and an outer tubular section forming the second one layer.

## Patentansprüche

1. Textiler Artikel (10) mit zwei oder mehr mindestens teilweise überlappenden Schichten, wobei eine erste der Schichten (11a) einen Abschnitt (15, 32) hat, der elektrisch leitendes Material umfasst, und eine zweite der Schichten (11b) einen Abschnitt (11b, 31) hat, der elektrisch isolierendes Material umfasst, wobei der Abschnitt (31) mit elektrisch isolierendem Material neben dem Abschnitt (32) mit elektrisch leitendem Material angeordnet ist, um den Abschnitt mit elektrisch leitendem Material mindestens teilweise abzudecken und somit elektrisch zu isolieren, **dadurch gekennzeichnet, dass** die erste (11a) und die zweite (11b) der Schichten auf einem gemeinsamen integralen Element (11) gebildet sind, das gefaltet wird, um einen überlappenden Bereich zu bilden, und **dadurch**, dass der Abschnitt (32), der elektrisch leitendes Material enthält, eine Elektrode (15) bildet, um elektrische Signale eines Trägers zu erkennen.

2. Textiler Artikel (10) nach Anspruch 1, wobei die erste und die zweite der Schichten einen gestrickten Aufbau haben.

3. Textiler Artikel (10) nach Anspruch 2, wobei die Schichten das Erzeugnis einer Rundstricktechnik sind.

4. Textiler Artikel nach Anspruch 3, wobei die erste und die zweite der Schichten aus einem Element (11) gebildet sind, das im Allgemeinen röhrenförmig ist, wobei das Element anschließend gefaltet wird, um einen Abschnitt (11a) des Elements in einem anderen Abschnitt (11b) des Elements zu verpacken, um einen röhrenförmigen Innenteil zu schaffen, der die erste Schicht bildet bzw. einen röhrenförmigen Außenteil, der die zweite Schicht bildet.

5. Textiler Artikel nach einem oder mehreren der Ansprüche 1 bis 4, wobei der Abschnitt (32) mit elektrisch leitendem Material Leiterbahnen (16) bildet.

6. Textiler Artikel nach einem oder mehreren der Ansprüche 1 bis 5, wobei ein textiler Artikel (10) ein Kleidungsstück, Kleidungszubehör, eine Gesundheitsüberwachungsvorrichtung, eine Gesundheitstherapievorrichtung, ein Sporttrainingsprodukt, eine tragbare Computeranordnung oder eine Komponente zur Verwendung in einem oder mehreren dieser Produkte ist.

7. Verfahren zum Schaffen eines textilen Artikels (10) mit zwei oder mehr mindestens teilweise überlappenden Schichten, wobei das genannte Verfahren die folgenden Schritte umfasst:
Schaffen einer ersten Schicht (11a) mit einem Abschnitt (15, 32), der elektrisch leitendes Material umfasst;
Schaffen einer zweiten Schicht (11b) mit einem Abschnitt, der elektrisch isolierendes Material umfasst;
Anordnen der genannten Schichten auf eine derartige Weise, dass sich der Abschnitt mit elektrisch isolierendem Material neben dem Abschnitt (32) mit elektrisch leitendem Material befindet, um den Abschnitt mit elektrisch leitendem Material mindestens teilweise abzudecken und somit elektrisch zu isolieren, **dadurch gekennzeichnet, dass** die erste (11a) und die zweite (11b) der Schichten gebildet werden, indem ein gemeinsames integrales Elements (11) gefaltet wird, um einen überlappenden Bereich zu bilden, und **dadurch**, dass der Abschnitt (32), der elektrisch leitendes Material enthält, eine Elektrode (15) bildet, um elektrische Signale eines Trägers zu erkennen.

8. Verfahren nach Anspruch 7, das den Schritt des Strickens der ersten und der zweiten der Schichten umfasst und **dadurch** die genannten Schichten schafft.

9. Verfahren nach Anspruch 8, wobei das Stricken ein Rundstricken ist.

10. Verfahren nach Anspruch 8 oder 9 und weiterhin die folgenden Schritte umfassend: Bilden der ersten und der zweiten der Schichten aus einem Element (11), das im Allgemeinen röhrenförmig ist; Falten des Elements, um einen Abschnitt (11a) des Elements in einem anderen Abschnitt (11b) des Elements zu verpacken, um einen röhrenförmigen Innenteil zu schaffen, der die erste Schicht bildet bzw. einen röhrenförmigen Außenteil, der die zweite Schicht bildet.

## Revendications

1. Article textile (10) comprenant deux ou plusieurs couches se recouvrant au moins partiellement, une première des couches (11a) comportant une partie (15, 32) qui comprend un matériau électriquement conducteur, et une deuxième des couches (11b) comportant une partie (11b, 31) qui comprend un matériau électriquement isolant, dans lequel la partie (31) comprenant un matériau électriquement isolant est disposée à côté de la partie (32) comprenant le matériau électriquement conducteur pour couvrir au moins partiellement et ainsi servir à isoler électriquement la partie comprenant un matériau électriquement conducteur, **caractérisé en ce que** la première (11a) et la deuxième (11b) des couches sont formées à partir d'un composant intégral commun (11) plié pour fournir une région de recouvrement, et **en ce que** la partie (32) qui comprend un matériau électriquement conducteur forme une électrode (15) pour détecter des signaux électriques d'un porteur.

2. Article textile (10) selon la revendication 1, dans lequel la première et la deuxième des couches sont de construction tricotée.

3. Article textile (10) selon la revendication 2, dans lequel les couches sont le produit d'une technique de tricotage circulaire.

4. Article textile selon la revendication 3, dans lequel la première et la deuxième des couches sont formées à partir d'un composant (11) qui est de forme généralement tubulaire, le composant étant par la suite plié pour rentrer une partie (11a) du composant dans une autre partie (11b) du composant afin de fournir, respectivement, une section tubulaire intérieure formant la première couche et une section tubulaire extérieure formant la deuxième couche.

5. Article textile selon l'une quelconque ou plusieurs des revendications 1 à 4, dans lequel la partie (32) qui comprend un matériau électriquement conducteur forme des pistes conductrices (16).

6. Article textile selon l'une quelconque ou plusieurs des revendications 1 à 5, dans lequel un article textile (10) est l'un parmi un vêtement, un accessoire d'habillement, un dispositif de surveillance de santé, un dispositif de soins de santé, un produit d'entraînement sportif, un agencement informatique vestimentaire ou un composant pour une utilisation dans un ou plusieurs de ces produits.

7. Procédé consistant à prévoir un article textile (10) comprenant deux ou plusieurs couches se recouvrant au moins partiellement, ledit procédé comprenant les étapes suivantes de :
fourniture d'une première couche (11a) comportant une partie (15, 32) qui comprend un matériau électriquement conducteur ;
fourniture d'une deuxième couche (11b) comportant une partie qui comprend un matériau électriquement isolant ;
agencement desdites couches de sorte que la partie comprenant le matériau électriquement isolant est disposé à côté de la partie (32) comprenant le matériau électriquement conducteur pour couvrir au moins en partie et ainsi servir à isoler électriquement la partie comprenant un matériau électriquement conducteur, **caractérisé en ce que** la première (11a) et la deuxième (11b) des couches sont formées par le pliage d'un composant intégral commun (11) pour fournir une région de recouvrement, et **en ce que** la partie (32) qui comprend un matériau électriquement conducteur forme une électrode (15) pour détecter des signaux électriques d'un porteur.

8. Procédé selon la revendication 7, comprenant l'étape de tricotage de la première et de la deuxième des couches, fournissant ainsi lesdites couches.

9. Procédé selon la revendication 8, dans lequel le tricotage est un tricotage circulaire.

10. Procédé selon la revendication 8 ou 9, et comprenant en outre les étapes de formation de la première et de la deuxième des couches à partir d'un composant (11) qui est de forme généralement tubulaire ; et de pliage des composants pour rentrer une partie (11a) du composant dans une autre partie (11b) du composant pour prévoir, respectivement, une section tubulaire intérieure formant la première couche et une section tubulaire extérieure formant la deuxième couche.
